# EUROPEAN PATENT APPLICATION

(11) **EP 3 264 085 A1**
(43) Date of publication of application: **03.01.2018**
(21) Application number: 16755662.0
(22) Date of filing: 25.02.2016
(51) Int. Cl.: G01N 33/531, G01N 33/53, G01N 33/543

(54) **IMMUNOASSAY METHOD AND ASSAY REAGENT USED IN SAID METHOD**

(30) Priority: 25.02.2015 JP 2015035926
(71) Applicant: Sekisui Medical Co., Ltd., Tokyo 103-0027 (JP)
(72) Inventor: KOBAYASHI, Koji, Tokyo 103-0027 (JP); MATSUMOTO, Takuji, Tokyo 103-0027 (JP); YAMAMOTO, Mitsuaki, Tokyo 103-0027 (JP)
(74) Representative: Hoffmann Eitle
(86) International application number: PCT/JP2016/055729
(87) International publication number: WO 2016/136918

(57) **Abstract**

A problem to be solved by the invention is to provide a method of avoiding an influence of hemoglobin in an immunological measurement method for an analyte in a biological sample. The problem is solved by reacting a sample suspected of containing an analyte with an antibody binding to the analyte in the presence of a polypeptide consisting of amino acids No. 419 to No. 607 of the amino acid sequence of DnaK, a heat shock protein (HSP), derived from E. coli as set forth in SEQ ID NO: 1 or a polypeptide having at least 90 % sequence identity with the polypeptide.

## Description

### TECHNICAL FIELD

The present invention relates to a method of avoiding an influence of hemoglobin in an immunological measurement and a reagent therefor.

### BACKGROUND ART

In recent years, measurement methods utilizing immunoreactions for measuring trace substances in biological samples are widely used. Immunological measurement methods include a number of methods such as a RIA method, an EIA method, a turbidimetric immunoassay, a latex agglutination method, a metal colloid aggregation method, and an immunochromatography method. Using whole blood as a sample has a problem that blood cell components such as hemoglobin and membrane components of blood cells mixed into the sample due to hemolysis affect optical detection systems, inhibit immunoreactions, or adsorb the analyte, thereby affecting the measurement. For example, it is widely known that hemoglobin is oxidized by a component etc. in a measurement reagent in the immunological measurement using an oxidation-reduction enzyme and a pigment-coloring system, thereby causing a change in the absorption wavelength of hemoglobin itself and resulting in an error of the measurement value of the analyte in the biological sample. In many cases, methods of avoiding the influence of hemoglobin by using various surfactants etc. are applied to immunological measurement using an oxidation-reduction enzyme and a pigment-coloring system.

On the other hand, a latex turbidimetric immunoassay is considered not to be affected by a change in the absorption wavelength of hemoglobin since the measurement wavelength is a long wavelength and does not overlap with the absorption wavelength of hemoglobin. However, actually, it is known that a measurement value may differ from the true value in the measurement of a sample containing hemoglobin.

Patent Document 1 describes a method of eliminating nonspecific agglutination considered to be attributable to the presence of native non-denatured hemoglobin (hemoglobin A₀) which is nonspecifically attracted to negatively-charged latex particles due to a net positive charge. By retaining pH of a reaction mixture to about 8.5 or more, the net charge of hemoglobin A₀ is neutralized to suppress the agglutination due to an electrostatic attractive force to the latex particles, so as to eliminate the nonspecific agglutination in a latex agglutination immunoassay performed on a whole blood sample.

In Patent Document 2, it is described that a reaction is performed in the presence of a macrocyclic compound such as calixarenes for the purpose of avoiding the influence of hemoglobin in an immunological method for measuring an analyte in a sample such as plasma derived from a living body. Particularly, it is described in an example that the influence of hemoglobin can be avoided by performing a latex agglutination method in the presence of calixarenes.

### CITATION LIST

### PATENT LITERATURE

Patent Document 1: Japanese Laid-Open Patent Publication No. H01-150857
Patent Document 2: WO 2010/001619

### SUMMARY OF INVENTION

### TECHNICAL PROBLEM

A problem to be solved by the present invention is to provide a method of avoiding an influence of hemoglobin in an immunological measurement method for an analyte in a biological sample.

### SOLUTION TO PROBLEM

[1] An immunoassay for an analyte in a sample containing hemoglobin, comprising: reacting an analyte in a sample suspected of being present in the sample containing hemoglobin with an antibody binding to the analyte in the presence of a polypeptide consisting of amino acids No. 419 to No. 607 of the amino acid sequence of DnaK, a heat shock protein (HSP), derived from E. coli as set forth in SEQ ID NO: 1 or a polypeptide having at least 90 % sequence identity with the polypeptide.
[2] The method according to item [1] above, wherein the analyte in a sample containing hemoglobin is reacted with an antibody binding to the analyte also in the presence of one or more buffer components selected from HEPES, Bis-Tris, TES, and Tris.
[3] The method according to item [1] or [2] above, wherein the antibody is immobilized on an insoluble carrier.
[4] The method according to item [3] above, wherein the insoluble carrier is latex particles or metal colloid particles.
[5] The method according to item [4] above, wherein a particle agglutination measurement method is utilized.
[6] The method according to any one of items [1] to [5] above, wherein the antibody is two or more monoclonal antibodies having recognition sites different from each other.
[7] The method according to item [6] above, wherein the two or more monoclonal antibodies having recognition sites different from each other are respectively immobilized on the latex particles, and wherein the analyte in the sample is detected by a latex turbidimetric immunoassay.
[8] The method according to any one of items [1] to [7] above, wherein the sample is urine, whole blood, serum, or plasma.
[9] The method according to any one of items [1] to [8] above, wherein the analyte is L-FABP (liver-type fatty acid binding protein).
[10] A method of avoiding an influence of hemoglobin in a method of detecting an analyte in a sample containing hemoglobin with an antibody binding to the analyte, the method comprising:
   reacting a sample suspected of containing the analyte with an antibody binding to the analyte
   in the presence of a polypeptide consisting of amino acids No. 419 to No. 607 of the amino acid sequence of DnaK, a heat shock protein (HSP), derived from E. coli as set forth in SEQ ID NO: 1 or a polypeptide having at least 90 % sequence identity with the polypeptide.
[11] The method according to item [10] above, wherein the analyte in a sample containing hemoglobin is reacted with an antibody binding to the analyte also in the presence of one or more buffer components selected from HEPES, Bis-Tris, TES, and Tris.
[12] The method according to item [10] or [11] above, wherein the antibody is immobilized on an insoluble carrier.
[13] The method according to item [12] above, wherein the insoluble carrier is latex particles or metal colloid particles.
[14] The method according to item [13] above, wherein a particle agglutination measurement method is utilized.
[15] The method according to any one of items [10] to [14] above, wherein the antibody is two or more monoclonal antibodies having recognition sites different from each other.
[16] The method according to item [15] above, wherein the two or more monoclonal antibodies having recognition sites different from each other are respectively immobilized on the latex particles, and wherein the analyte in the sample is detected by a latex turbidimetric immunoassay.
[17] The method according to any one of items [10] to [16] above, wherein the sample is urine, whole blood, serum, or plasma.
[18] The method according to any one of items [10] to [17] above, wherein the analyte is L-FABP (liver-type fatty acid binding protein).

### ADVANTAGEOUS EFFECTS OF INVENTION

According to the present invention, the influence of hemoglobin in a biological sample can be avoided and, therefore, accurate measurement can be performed even if the sample contains hemoglobin. The present invention provides an immunoassay and an immunoassay reagent enabling accurate measurement of an analyte in a sample containing hemoglobin, and a method of avoiding an influence of hemoglobin in an immunoassay for an analyte in a sample containing hemoglobin.

### DESCRIPTION OF EMBODIMENTS

### (Sample)

Examples of the biological sample used in the present invention include urine, blood (whole blood, plasma, or serum), tissues of kidney, heart, liver, etc., and extract from the tissues, etc. Any samples are usable as long as the samples are suspected of containing an analyte, including a sample derived from a healthy subject, a sample derived from a patient, a sample derived from a person suspected of having a disease, etc.

### (Substance to Be Measured)

The analyte subjected to measurement according to the present invention is not particularly limited.

The present invention will hereinafter be described by using urine as a sample and liver-type fatty acid binding protein (L-FABP) as an analyte, by way of example.

### (Anti-L-FABP Antibody)

For the anti-L-FABP antibody used in the present invention, a native L-FABP purified from organs, cells, body fluid, etc. can be prepared as an immunogen (antigen). L-FABP is mainly distributed in the liver or the kidney and therefore can be purified and isolated from these organs etc. Additionally, it is known that L-FABP is highly homologous among humans, mice, pigs, cows, and rats and has a homology of 90% or more at the amino acid level and, therefore, for example, mouse L-FABP can be used as an antigen for acquiring an antibody binding to human L-FABP.

Purification of native L-FABP can be performed in accordance with a method described in Kelvin et al. (J. Biol. Chem., vol.263, pp.15762-15768, 1988) etc. In particular, after homogenizing an excised organ, a cytoplasmic fraction acquired by ultracentrifugation is fractionated by gel filtration, anion exchange chromatography, etc., and the fraction containing L-FABP is selected by using a molecular weight or fatty acid binding activity as an index, isolated, and purified. The selected fraction is subjected to SDS-polyacrylamide electrophoresis to confirm that the purified protein forms a single band and is further purified if needed. For the purified protein, the amino-acid composition and the N-terminal amino-acid sequence can be determined and compared with the reported composition and sequence so as to confirm that the protein is the intended molecular species.

L-FABP used as an antigen may be a recombinant protein produced by a genetic engineering technique. Since the amino acid sequence and the gene sequence of L-FABP are already reported (Veerkamp and Maatman, Prog. Lipid Res., vol.34, pp. 17-52, 1995), for example, a primer can be designed based on these sequences for cloning of cDNA from an appropriate cDNA library etc. by a PCR (polymerase chain reaction) method. This cDNA can be used for performing gene recombination so as to prepare recombinant L-FABP. Additionally, a fragment of L-FABP or a synthetic peptide etc. having a partial sequence thereof can be bound to a carrier polymeric substance (BSA, hemocyanin, etc.) as needed and used as the antigen.

An antibody specifically binding to L-FABP may be any of antisera, polyclonal antibodies, monoclonal antibodies, etc.

The antibody preferably has a high specificity and, for example, in the case of an anti-L-FABP antibody, desirably, the antibody is substantially not cross-reactive with H-FABP. To acquire an antibody with a higher specificity, a highly purified and highly pure antigen is desirably used. When an antibody is prepared, a warm-blooded animal other than human is immunized by inoculating the purified antigen prepared as described above. Examples of the warm-blooded animal to be immunized other than human include mammals (rabbits, sheep, rats, mice, guinea pigs, horses, pigs, etc.) and birds (chickens, ducks, geese etc.). In the case of rabbits, for example, about 100 µg to 1 mg of the antigen emulsified in about 1 mL of saline and Freund's complete adjuvant is inoculated subcutaneously in the dorsum or the palm of a hind foot and, from the second time, the adjuvant is replaced with Freund's incomplete adjuvant, the antigen is inoculated three to eight times at intervals of two to four weeks for immunization, and the antibody is produced about 7 to 12 days after the final inoculation and used. In the case of mice, 10 to 30 µg/animal of the antigen is usually inoculated subcutaneously, intraperitoneally, or intravenously three to eight times at intervals of about two weeks for immunization, so as to use the antibody produced about two to four days after the final inoculation.

The polyclonal antibodies can be prepared by collecting blood from the animal immunized as described above, separating serum (antiserum), and recovering an Ig fraction from the acquired antiserum. For example, polyclonal IgG can be acquired by recovering an IgG fraction from the antiserum by affinity chromatography using a Protein G column etc.

The monoclonal antibodies are produced by a hybridoma acquired by fusing antibody-producing cells collected from an immunized animal with immortalized cells. Mice and rats are preferably used as immunized animals for the monoclonal antibodies. The hybridoma can be produced in accordance with the method of Kohler and Milstein (Kohler and Milstein, Nature, vol.256, pp.495-887, 1975) as follows. Antibody-producing cells (such as splenocytes or lymph node cells) from an animal immunized as described above are collected and fused with appropriate immortalized cells. For example, cell lines of myeloma cells (NSI-Ag 4/1, Sp2/O-Agl4, etc.) are preferably used as immortalized cells. The myeloma cells are preferably nonsecretory cells not producing antibodies or immunoglobulin H or L chains by themselves. The myeloma cells preferably have a selection marker so that unfused myeloma cells and fused hybridomas may be screened in a selection medium. For example, for the selection marker, cell lines having 8-azaguanine resistance (hypoxanthine-guanine-phosphoribosyltransferase deficiency), thymidine kinase deficiency, etc. are often used. The cell fusion is performed by adding an appropriate fusion promoter such as polyethylene glycol. The cell fusion is preferably performed at a ratio of about 10 antibody-producing cells per immortalized cell, and can preferably be performed at a cell density of about 10⁶ cells/mL of the antibody-producing cells.

The cells subjected to the fusion treatment are appropriately diluted and then cultured in a selection medium for one to two weeks. For example, when myeloma cells resistant to 8-azaguanine are used, the unfused myeloma cells cultured in the HAT (hypoxanthine, aminopterin, thymidine) medium die, and the unfused antibody-producing cells cultured in the HAT (hypoxanthine, aminopterin, thymidine) medium also die because of limited division cycle; however, only the fused cells can continue to undergo division and survive in the selection medium. After culturing in the selection medium, the supernatant is subjected to, for example, ELISA with an antigen immobilized on a solid phase, so as to detect the presence/absence of the intended antibody, and cloning can be performed by a limiting dilution method to select a hybridoma producing a monoclonal antibody recognizing the intended antigen. At the time of selection, the hybridoma can be selected that produces the monoclonal antibody having desired properties such as antibody titer, antibody class, subclass, affinity for antigen, specificity, epitope, etc. IgG is generally preferable as the class of monoclonal antibody.

The monoclonal-antibody-producing hybridoma is intraperitoneally implanted in an animal of the same species as the animal used for immunization and, after elapse of a certain period, the ascites can be collected from the animal to isolate the intended monoclonal antibody. Alternatively, the hybridoma can be cultured in an appropriate animal cell culture medium, and the monoclonal antibody can be isolated from the culture solution. Once the intended hybridoma is acquired, the gene encoding the monoclonal antibody can be acquired from the hybridoma to express and produce the intended monoclonal antibody in an appropriate host (e.g., silkworm, etc.) by a common gene recombination technique. Separation and purification of the antibody can be performed in accordance with, for example, a usual purification method combining ammonium sulfate precipitation, gel chromatography, ion exchange chromatography, affinity chromatography, etc. as needed.

The anti-L-FABP antibody used in the present invention may be a known antibody or an antibody to be developed in the future. Although not particularly limited, usable commercially available anti-L-FABP antibodies include C-4 (Catalog No. sc-374537), F-9 (Catalog No. sc-271591) of Santa Cruz Biotechnology, 328607 (Catalog No. MAB2964) of R&D systems, L2B10 (Catalog No. HA 2049-IA) of Hycult biotech, 2G4 (Catalog No. LS-B3001) of Lifespan Biosciences, etc.

"Antibody" in the present invention includes not only intact immunoglobulin molecules but also antibody fragments or antibody derivatives having antigen binding abilities known in the art, such as Fab, Fab'₂, CDR, a humanized antibody, a multifunctional antibody, and a single chain antibody (ScFv).

### (Detection)

The method of the present invention for detecting L-FABP using an anti-L-FABP antibody is an immunological measurement method. More specifically, examples thereof include, but not limited to, a particle immunoagglutination measurement method such as a latex turbidimetric immunoassay (LTIA), ELISA, a chemiluminescence detection method, and immunochromatography (lateral-flow type, flow-through type). Among them, an immunological measurement method not including a step for B/F separation (homogeneous immunoassay method) is more preferable.

It is noted that when LTIA is described as a measurement method in this description, the detection method thereof may be achieved by using any of known detection methods such as measurement of change in transmitted light (absorbance), measurement of change in scattered light, and measurement of change in particle diameter.

Furthermore, the term "detection" or "measurement" must be construed in the broadest sense including the proof of the presence and/or the quantification of L-FABP, and must not be construed in a limited manner.

### (Insoluble Carrier)

An insoluble carrier used in the present invention can be an insoluble carrier made of a polymeric base material such as polystyrene resin, an inorganic base material such as glass, a polysaccharide base material such as cellulose and agarose, etc. and is not particularly limited in terms of the shape thereof, and any shapes can be selected in accordance with the measurement method to be adopted, including a bead or particle shape (e.g., latex particles, metal colloid particles), a plate or sheet shape (e.g., a porous membrane, an immunoplate), a tubular shape (e.g., a test tube), etc.

Examples of the particles include latex particles mainly composed of polystyrene generally used in the particle immunoagglutination measurement method as well as particles containing a styrene-butadiene copolymer, a (meth)acrylic acid ester polymer, etc. as a base material. Particles made of metal colloid, gelatin, liposome, microcapsule, silica, alumina, carbon black, metallic compound, metal, ceramics, or magnetic material are also usable. For the carrier particles used in the present invention, one and the same kind of material or two or more kinds of materials can be used.

The particle diameter of the carrier particles is preferably 0.15 to 0.45 µm, more preferably 0.2 to 0.4 µm. Two or more kinds of the carrier particles different in average particle diameter can be used in combination.

The porous membrane can be a known membrane and can be made of any material. Examples of the material of the porous membrane include, but not limited to, polyethylene, polyethylene terephthalate, nylons, glass, polysaccharides such as cellulose and cellulose derivatives, ceramics, etc. Specifically, the examples include glass fiber filter paper, cellulose filter paper, etc. sold by Millipore, Toyo Roshi, Whatman, etc.

The plate (immunoplate) can be a known plate and can be made of any material. Examples of the material of the plate include, but not limited to, synthetic polymeric compounds such as vinyl chloride, polyethylene, polystyrene, polypropylene, and polyolefin elastomer, as well as glass etc.

### (Immobilization of Antibody to Insoluble Carrier)

A method for immobilizing an anti-L-FABP antibody on an insoluble carrier is not particularly limited, and any known method can be used. If an anti-L-FABP antibody is immobilized on particles, this is achieved by using, for example, a physical adsorption method using physical adsorption caused by mixing particles and the antibody, or a chemical binding method using a coupling agent such as carbodiimide to chemically bind a carboxy or an amino group on the particle surface to an antibody molecule. The antibody molecules may be immobilized on the particles via spacer molecules. Furthermore, after binding the antibody to another protein such as albumin by using the chemical binding method, the protein may physically or chemically be immobilized on the particles.

If an anti-L-FABP antibody is immobilized on a porous membrane, the antibody can be immobilized, for example, by applying a certain amount of a solution containing the antibody into a shape of a line, a dot, a specific symbol such as + to the porous membrane.

In this description, the "insoluble carrier" is referred to as a "solid phase", and allowing, or a state of allowing, the insoluble carrier to physically or chemically support an antigen or an antibody is referred to as "immobilization", "immobilized", "solid-phased", "sensitization", or "adsorption" in some cases.

### (Labeled Antibody)

Examples of a labeling substance for labeling the antibody include, for example, an enzyme, a fluorescent substance, a chemiluminescent substance, biotin, avidin, a radioactive isotope, gold colloid particles, or colored latex particles. A method of binding the labeling substance and the antibody can be methods such as a glutaraldehyde method, a maleimide method, a pyridyl disulfide method, or a periodic acid method available to those skilled in the art. Both the labeling substance and the binding method are not limited to those described above and any known methods can be used.

With regard to the detection of the label, for example, when an enzyme such as peroxidase or alkaline phosphatase is used as the labeling substance, the enzymatic activity can be measured by using a specific substrate of the enzyme (e.g., 1,2-phenylenediamine or 3,3',5,5'-tetramethylbenzidine when the enzyme is horseradish peroxidase, or p-nitrophenyl phosphate in the case of alkaline phosphatase) and, when biotin is used as the labeling substance, avidin labeled at least with a labeling substance other than biotin is typically reacted therewith.

### (BPF)

A polypeptide consisting of amino acids No. 419 to No. 607 of the amino acid sequence of DnaK, a heat shock protein (HSP), derived from E. coli (also referred to as Blocking Peptide Fragment; hereinafter sometimes also referred to as "BPF") is disclosed as a novel substance for blocking in an immunological measurement method in WO 2005/003155 and Polymer Preprints, Japan Vol. 55, No. 2, 5211-5212 (2006), and is also commercially available.

The BPF used for a method of the present invention etc. can be prepared in accordance with the description of WO 2005/003155. Alternatively, a commercially available product (manufactured by TOYOBO Co., Ltd., Catalog No. BPF-301) may be used.

The working concentration of BPF in the method etc. of the present invention is preferably 0.05 to 5 % (w/v%) in terms of a concentration in the measuring reagent or specimen diluent and can be more preferably 0.075 to 5 %, further preferably 0.1 to 3 %, for example. Those skilled in the art can experimentally determine the optimum concentration of BPF in consideration of the properties and concentration (amount) of a protein to be preserved.

For example, when BPF-301 having a molecular weight of about 22,000 is used, the concentration can be preferably 0.022 mmol/L to 2.27 mmol/L, more preferably 0.034 mmol/L to 2.27 mmol/L, further preferably 0.045 mmol/L to 1.36 mmol/L.

### (Method of Bringing Sample Suspected of Containing L-FABP, Anti-L-FABP Antibody, and BPF into Contact with Each Other)

The step of bringing a sample suspected of containing L-FABP, an anti-L-FABP antibody, and BPF into contact with each other may be achieved by using any method as long as a step of bringing the sample suspected of containing L-FABP and BPF into contact with each other is followed by a step of bringing the sample into contact with the anti-L-FABP antibody.

For example, the method of bringing the sample suspected of containing L-FABP into contact with the anti-L-FABP antibody and BPF can be, for example, a method of mixing a liquid reagent containing particles having the immobilized anti-L-FABP antibody and BPF with the sample. Another method can be a method of supplying the sample suspected of containing L-FABP to an insoluble carrier such as a porous membrane infiltrated with BPF so that the contact occurs. Those skilled in the art can appropriately achieve the setting in consideration of the configuration of the measurement reagent etc.

Furthermore, L-FABP in the sample is by a known appropriate method brought into contact with an anti-L-FABP antibody immobilized on an insoluble carrier, after, or at the same time as, the contact with BPF.

### (Buffer Agent)

A compound represented by following General Formula [I] can be exemplified as a buffer agent comprising a compound having a group represented by following [Chem 1] in a molecule of the present invention or a salt thereof.

### General formula [I]

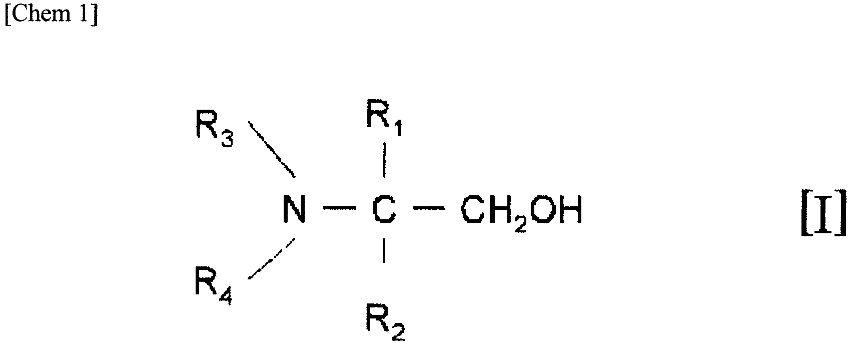

R₁ and R₂ in General Formula [I] may be the same as or different from each other and examples can include a hydrogen atom, an alkyl group, a hydroxyalkyl group, a di(hydroxyalkyl) alkyl group, a tri(hydroxyalkyl) group, etc. Examples of the alkyl group, the hydroxyalkyl group, the di(hydroxyalkyl) alkyl group, and the tri(hydroxyalkyl) alkyl group described above can include an alkyl group having the carbon number of 1 to 6 on a straight chain or a branched chain, and the alkyl group having the carbon number of 1 to 6 on a straight chain or a branched chain can specifically be exemplified by a methyl group, an ethyl group, a propyl group, a butyl group, an isobutyl group, a sec-butyl group, a tert-butyl group, a pentyl group, a hexyl group, etc.

R₃ and R₄ in General Formula [I] may be the same as or different from each other and examples can include a hydrogen atom, an alkyl group, a hydroxyalkyl group, a di(hydroxyalkyl) alkyl group, a tri(hydroxyalkyl) alkyl group, a carboxyalkyl group, a di(carboxyalkyl) alkyl group, a tri(carboxyalkyl) alkyl group, a sulfoalkyl group, a di(sulfoalkyl) alkyl group, a tri(sulfoalkyl) alkyl group, a sulfo-hydroxy-alkyl group, a di(sulfo-hydroxy-alkyl) alkyl group, a tri(sulfo-hydroxy-alkyl) alkyl group, etc.

In the alkyl group, the hydroxyalkyl group, the di(hydroxyalkyl) alkyl group, the tri(hydroxyalkyl) alkyl group, the carboxyalkyl group, the di(carboxyalkyl) alkyl group, the tri(carboxyalkyl) alkyl group, the sulfoalkyl group, the di(sulfoalkyl) alkyl group, the tri(sulfoalkyl) alkyl group, the sulfo-hydroxy-alkyl group, the di(sulfo-hydroxy-alkyl) alkyl group, and the tri(sulfo-hydroxy-alkyl) alkyl group described above, the alkyl group can include for example an alkyl group having the carbon number of 1 to 6 on a straight chain or a branched chain, and the alkyl group having the carbon number of 1 to 6 on a straight chain or a branched chain can specifically be exemplified by a methyl group, an ethyl group, a propyl group, a butyl group, an isobutyl group, a sec-butyl group, a tert-butyl group, a pentyl group, a hexyl group, etc.

Examples of R₃ and R₄ in General Formula [I] also include a group in which R₃ and R₄ form a cyclic structure together with a nitrogen atom to form a substituted or unsubstituted piperazinyl group, a substituted or unsubstituted morpholino group, or a substituted or unsubstituted piperidino group, and examples of the substituent in the substituted piperazinyl group, the substituted morpholino group, and the substituted piperidino group include an alkyl group, a hydroxyalkyl group, a di(hydroxyalkyl) alkyl group, a tri(hydroxyalkyl) alkyl group, a carboxyalkyl group, a di(carboxyalkyl) alkyl group, a tri(carboxyalkyl) alkyl group, a sulfoalkyl group, a di(sulfoalkyl) alkyl group, a tri(sulfoalkyl) alkyl group, a sulfo-hydroxy-alkyl group, a di(sulfo-hydroxy-alkyl) alkyl group, a tri(sulfo-hydroxy-alkyl), etc.

In the alkyl group, the hydroxyalkyl group, the di(hydroxyalkyl) alkyl group, the tri(hydroxyalkyl) alkyl group, the carboxyalkyl group, the di(carboxyalkyl) alkyl group, the tri(carboxyalkyl) alkyl group, the sulfoalkyl group, the di(sulfoalkyl) alkyl group, the tri(sulfoalkyl) alkyl group, the sulfo-hydroxy-alkyl group, the di(sulfo-hydroxy-alkyl) alkyl group, and the tri(sulfo-hydroxy-alkyl) described above, the alkyl group can include for example the alkyl group having the carbon number of 1 to 6 on a straight chain or a branched chain described above.

Specifically, the hydroxyalkyl group can be exemplified by a hydroxymethyl group, 2-hydroxyethyl group, 2-hydroxypropyl group, 2-hydroxybutyl group, 2-hydroxypentyl group, 2-hydroxyhexyl group, etc.; the di(hydroxyalkyl) alkyl group can be exemplified by a di(hydroxymethyl) methyl group, a di(2-hydroxyethyl) methyl group, etc.; the tri(hydroxyalkyl) alkyl group can be exemplified by a tri(hydroxymethyl) methyl group, a tri(2-hydroxyethyl) methyl group, etc.; the carboxyalkyl group can be exemplified by a carboxymethyl group, a 2-carboxyethyl group, etc.; the di(carboxyalkyl) alkyl group can be exemplified by a di(carboxymethyl) methyl group, a di(2-carboxyethyl) methyl group, etc.; and the tri(carboxyalkyl) alkyl group can be exemplified by a tri(carboxymethyl) methyl group, a tri(2-carboxyethyl) methyl group, etc.

Specifically, the sulfoalkyl group can be exemplified by a sulfomethyl group, a 2-sulfoethyl group, etc.; the di(sulfoalkyl) alkyl group can be exemplified by a di(sulfomethyl) methyl group, a di(2-sulfoethyl) methyl group, etc.; the tri(sulfoalkyl) alkyl group can be exemplified by a tri(sulfomethyl) methyl group, a tri(2-sulfoethyl) methyl group, etc.; the sulfo-hydroxy-alkyl group can be exemplified by a 2-hydroxy-3-sulfopropyl group, a 3-hydroxy-4-sulfopropyl group, etc.; the di(sulfo-hydroxy-alkyl) alkyl group can be exemplified by a di(2-hydroxy-3-sulfopropyl) methyl group, a di(3-hydroxy-4-sulfopropyl) methyl group, etc.; and the tri(sulfo-hydroxy-alkyl) alkyl group can be exemplified by a tri(2-hydroxy-3-sulfopropyl) methyl group, a tri(3-hydroxy-4-sulfopropyl) methyl group, etc.

Specific examples of the buffer agent used in the present invention include Bicine (CAS No. 150-25-4), BES (CAS No. 10191-18-1), BisTris (CAS No. 6976-37-0) DIPSO (CAS No. 68399-80-4), HEPES (CAS No. 7365-45-9), HEPPS (CAS No. 16052-06-5), HEPPSO Hydrate (CAS No. 68399-78-0), Tricine (CAS No. 5704-04-1), TES (CAS No. 7365-44-8), TAP (CAS No. 29915-38-6), TAPSO (CAS No. 68399-81-5), Bis Tris propane (CAS No. 64431-96-5), Tris hydrochloride (CAS No. 1185-53-1), Tris base (CAS No. 77-86-1), TES sodium hydrate (CAS No. 70331-82-7), etc. Particularly, BisTris, HEPES, TES, and Tris are preferred.

### (Measurement Kit)

Constituents of a measurement kit provided according to the present invention are not particularly limited as long as L-FABP can immunologically be measured. The measurement kit will hereinafter be described by taking sandwich ELISA, immunochromatography, and LTIA as an example.

### <Sandwich ELISA>

In the case of the sandwich ELISA, the measurement kit includes at least (a) an insoluble carrier having an anti-L-FABP antibody of the present invention immobilized thereon and (b) an antibody labeled with a labeling substance and having a property of reacting with L-FABP. In this case, the insoluble carrier is preferably a plate (immunoplate), and the labeling substance can appropriately be selected and used.

The antibody immobilized on the insoluble carrier captures L-FABP in the sample and forms a complex on the insoluble carrier. The antibody labeled with the labeling substance binds to the captured L-FABP and forms a sandwich with the complex described above. L-FABP in the sample can be measured by measuring an amount of the labeling substance with a method corresponding to the labeling substance. Specific methods such as a method of immobilizing the antibody on the insoluble carrier and a method of binding the antibody and the labeling substance can be achieved by using methods well known to those skilled in the art without particular limitation. Although either a homogeneous measurement method or a heterogeneous measurement method can be configured in the case of this configuration, the homogeneous measurement method is more preferable.

For example, BPF can be added, for example, to a sample diluent or a solution for an antigen-antibody reaction and thereby can be brought into contact with L-FABP in the sample.

### <Immunochromatography>

Typical immunochromatography is configured by using a test strip equipped with "1. a sample-supply portion", "2. a portion of retaining a labeled antibody (a labeled antibody-retaining portion)", and "3. a portion of immobilizing an antibody for capturing a complex formed by the labeled antibody and the L-FABP antibody (a capture-antibody portion)" in the order in a direction of development of a solution containing a sample on a sheet-like insoluble carrier such as a porous membrane, such that the sample solution continuously moves due to capillarity. In the case of immunochromatography, the measurement kit at least includes the test strip as described above.

Specifically, when a predetermined amount of a sample containing L-FABP is added to the sample-supply portion, the sample enters the labeled- retaining portion due to capillarity, and L-FABP and the labeled antibody bind together to form a complex. When the complex developed through the membrane enters the capture-antibody portion, the complex is captured by the antibody (capture antibody) immobilized on the membrane to form a ternary complex of [the capture antibody]-[L-FABP]-[the labeled antibody]. The label can be detected by an arbitrary method (e.g., an agglutination image in the case of a label that can be made visible such as gold colloid particles, or a coloring reaction due to addition of a substrate in the case of an enzyme), so as to detect the presence of L-FABP.

For example, BPF can preliminarily be added to a sample diluent etc. or can preliminarily be contained in the sample-supply portion or the labeled- retaining portion and thereby can be brought into contact with L-FABP in the sample.

### <Latex Turbidimetric Immunoassay>

In the case of latex turbidimetric immunoassay, the measurement kit includes at least latex particles having an antibody immobilized thereon. For the antibody used in latex turbidimetric immunoassay, any combination of "two monoclonal antibodies having different recognition sites for antigens", "polyclonal antibodies", or "monoclonal antibody and polyclonal antibody" can be used. In this case, the latex particles are the insoluble carrier having an antibody immobilized thereon and the labeling substance at the same time.

The latex particles used for these measurement reagents can appropriately be selected in terms of particle diameter and material so as to acquire desired performance such as improved sensitivity. The latex particles may be any particles suitable for supporting the antibody. For example, the particles may contain polystyrene, a styrene-sulfonic acid (salt) copolymer, a styrene-methacrylic acid copolymer, an acrylonitrile-butadiene-styrene copolymer, a vinyl chloride- acrylic acid ester copolymer, a vinyl acetate-acrylic acid ester copolymer, etc., as a base material. Although the shape of the latex particles is not particularly limited, preferably, the average particle diameter is sufficiently large so that aggregates generated as a result of agglutination reaction between the antibody on the latex particle surfaces and L-FABP can be detected with the naked eye or optically. Particles made of material such as metal colloid, gelatin, liposome, microcapsule, silica, alumina, carbon black, metal compound, metal, ceramics, or magnetic material can be used instead of the latex particles.

A typical measurement kit for LTIA used in clinical examination is usually provided in a form of a first reagent and a second reagent. BPF and the latex particles having the antibody immobilized thereon can be contained in the first reagent or the second reagent. Although it is generally preferable that the latex particles having the antibody immobilized thereon be contained in the second reagent, the particles can be contained in the first reagent. Alternatively, the latex particles can be contained in both the first reagent and the second reagent.

The kit of the present invention also includes saccharides, proteins, etc. as needed for the purpose of improving measurement sensitivity and suppressing nonspecific reaction. Examples thereof include components promoting antigen-antibody reactions (polymeric compounds such as polyethylene glycol, polyvinyl pyrrolidone, phospholipid polymers, etc.), proteins and peptides (albumin, casein, etc.), amino acids, sugars (sucrose, cyclodextrin, etc.), and preservatives (sodium azide, ProClin 300, etc.).

Although native L-FABP derived from tissues such as liver and kidney can be used as a standard substance (L-FABP standard substance) in sample measurement, the standard substance may be a recombinant protein produced by a genetic engineering technique. Since the amino acid sequence and the gene sequence of L-FABP have already been reported (Veerkamp and Maatman, Prog. Lipid Res., vol.34, pp. 17-52, 1995), for example, a primer can be designed based on these sequences for cloning of cDNA from an appropriate cDNA library etc. by a PCR (polymerase chain reaction) method. This cDNA can be used for preparing recombinant L-FABP by gene recombination techniques. For the standard substance, it is more preferable to use the recombinant protein with stable structure.

### EXAMPLES

Examples of the present invention will hereinafter be described to more specifically describe the present invention; however, the present invention is not limited thereto and can variously be applied without departing from the technical idea of the present invention.

### (Anti-L-FABP Antibody-Immobilized Latex Particle Suspension)

### (1) Preparation of Clone L Antibody-Immobilized Latex Particle Suspension

To 13 mL of a 20 mmol/L Tris buffer solution (pH 8.5) containing 0.36 mg/mL of anti-L-FABP antibody Clone L (manufactured by CMIC HOLDINGS Co., Ltd.), 13 mL of a 1% latex particle (manufactured by SEKISUI CHEMICAL CO., LTD.) suspension having an average particle diameter of 0.21 µm was added and stirred at 4 °C for two hours. This was followed by addition of 13 mL of a 20 mmol/L Tris buffer solution (pH 8.5) containing 0.5 % BSA and stirring at 4 °C for one hour. Subsequently, dialysis with a 5 mmol/L MOPS buffer solution (pH 7.0) was performed to acquire a Clone L antibody-immobilized latex particle suspension.

### (2) Preparation of Clone 1 Antibody-Immobilized Latex Particle Suspension

To 8 mL of a 5 mmol/L Tris buffer solution (pH 7.5) containing 0.54 mg/mL of anti-L-FABP antibody Clone 1 (manufactured by CMIC HOLDINGS Co., Ltd.), 8 mL of a 1 % latex particle (manufactured by SEKISUI CHEMICAL CO., LTD.) suspension having an average particle diameter of 0.32 µm was added and stirred at 4 °C for two hours. This was followed by addition of 8 mL of a 5 mmol/L Tris buffer solution (pH 7.5) containing 0.5 % BSA and stirring at 4 °C for one hour. Subsequently, dialysis with a 5 mmol/L MOPS buffer solution (pH 7.0) was performed to acquire a Clone 1 antibody-immobilized latex particle suspension.

### (L-FABP Standard Substance)

The L-FABP standard substance was acquired by gene recombination as described in Japanese Laid-Open Patent Publication No. H11-242026.

### (L-FABP Reference Measurement Method: Reference Method)

An ELISA-based in vitro diagnostic product (Renapro (registered trademark) L-FABP test TMB) was used for a reference method.

### (First Reagent: Also Serving as Standard Substance Diluent)

300 mmol/L HEPES buffer solution (pH 7.0)
BPF at each concentration (manufactured by TOYOBO Co., Ltd., BPF-103)
100 mmol/L NaCl
390 mmol/L benzamidine hydrochloride
0.4 % Lipidure-BL103

### (Second Reagent)

5 mmol/L MOPS buffer solution (pH 7.0)
3.75 Abs/mL Clone L antibody-immobilized latex particle suspension^{(*)}
1.25 Abs/mL Clone 1 antibody-immobilized latex particle suspension^{(*)}
(*) Abs denotes the absorbance at 280 nm.

### (Standard Solution)

The L-FABP standard substance was adjusted to a desired concentration by using the standard substance diluent and was used as a standard solution.

### (Frozen-Thawed Urine)

Partial urine frozen and stored at -30 °C after collection was thawed only once and used for measurement. L-FABP in the frozen-thawed urine measured in advance by the reference method was 10 ng/mL.

### (Specimen)

Interference Check A Plus (manufactured by SYSMEX CORPORATION) was adjusted to set the hemoglobin concentration to 0 to 1000 mg/dL. The adjusted Interference Check A Plus and the frozen-thawed urine were mixed in equal amounts to acquire a specimen in which hemoglobin coexisted at each concentration.

### (Measurement Conditions of LTIA)

(1) Analyzing device: Hitachi 7170 Automatic Analyzer (manufactured by Hitachi High-Technologies Corporation)
(2) Sample amount and reagent amounts: 3 µL of sample, 150 µL of the first reagent, 50 µL of the second reagent
(3) Reaction time (reaction temperature): 5 minutes (37 °C) for the first reagent, 5 minutes (37 °C) for the second reagent
(4) Photometric point and photometric object: absorbance changes between immediately after addition of the second reagent and 5 minutes after the addition

### [Examples 1 to 4] Avoidance of Influence of Hemoglobin by BPF

The effect of avoiding an influence of hemoglobin by BPF was confirmed.

### 1. Operation

### (1) Preparation of Calibration Curve

The standard solution was employed as a sample and L-FABP in the sample was measured by using the first reagent and the second reagent. From the measured absorbance, an absorbance of a 0 ng/mL L-FABP sample (blank absorbance) was subtracted to calculate the net absorbance. A calibration curve was prepared by plotting the L-FABP concentration on the x axis and the net absorbance on the y axis.

### (2) Examples 1 to 4

L-FABP in the specimen was measured by using the first reagent containing BPF at the concentration in Table 1 and the second reagent.

**[Table 1]**

| | BPF conc. |
|---|---|
| Ex.1 | 0.1% |
| Ex.2 | 0.5% |
| Ex.3 | 1.0% |
| Ex.4 | 2.0% |

| | |
|---|---|
| Ex.: Example conc.: concentration | |

### (3) Control Example

L-FABP in the sample was measured by using the first reagent not containing BPF and the second reagent.

### 2. Results

The L-FABP concentration was calculated by using the calibration curve of (1) from the absorbance measured in Examples 1 to 4 and Control Example. The measurement value calculated at each hemoglobin concentration was divided by the measurement value at 0 mg/dL of hemoglobin to obtain a ratio (%) shown in Table 2.

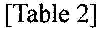

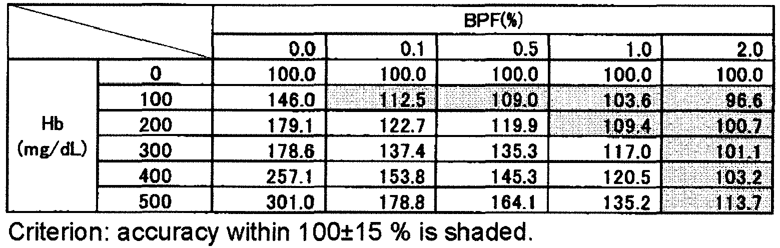

When BPF was not added, the ratio was already near 150 % in coexistence with 100 mg/dL hemoglobin. On the other hand, when BPF was added, the ratio was reduced depending on the concentration of BPF, and variations were within 15 % in shaded portions as shown in Table 2. Thus, variations were within ±15 % at the hemoglobin concentration of 100 mg/dL, and it was confirmed that the influence of hemoglobin can be avoided. It was also confirmed that at the hemoglobin concentration of 200 to 500 mg/dL, the ratio is reduced depending on the concentration of BPF.

For Examples 5 to 9, measurement was performed by using the following materials under the following conditions.

### (Anti-L-FABP Antibody-Immobilized Latex Particle Suspension)

### (1) Preparation of Clone L Antibody-Immobilized Latex Particle Suspension

To 13 mL of a 20 mmol/L Tris buffer solution (pH 8.5) containing 0.36 mg/mL of anti-L-FABP antibody Clone L (manufactured by CMIC HOLDINGS Co., Ltd.), 13 mL of a 1% latex particle (manufactured by SEKISUI CHEMICAL CO., LTD.) suspension having an average particle diameter of 0.27 µm was added and stirred at 4 °C for two hours. This was followed by addition of 13 mL of a 20 mmol/L Tris buffer solution (pH 8.5) containing 0.5 % BSA and stirring at 4 °C for one hour. Subsequently, dialysis with a 5 mmol/L MOPS buffer solution (pH 7.0) was performed to acquire a Clone L antibody-immobilized latex particle suspension.

### (2) Preparation of Clone 1 Antibody-Immobilized Latex Particle Suspension

To 8 mL of a 5 mmol/L Tris buffer solution (pH 7.5) containing 0.54 mg/mL of anti-L-FABP antibody Clone 1 (manufactured by CMIC HOLDINGS Co., Ltd.), 8 mL of a 1 % latex particle (manufactured by SEKISUI CHEMICAL CO., LTD.) suspension having an average particle diameter of 0.25 µm was added and stirred at 4 °C for two hours. This was followed by addition of 8 mL of a 5 mmol/L Tris buffer solution (pH 7.5) containing 0.5 % BSA and stirring at 4 °C for one hour. Subsequently, dialysis with a 5 mmol/L MOPS buffer solution (pH 7.0) was performed to acquire a Clone 1 antibody-immobilized latex particle suspension.

### (L-FABP Standard Substance)

The L-FABP standard substance was acquired by gene recombination as described in Patent Document 1.

### (L-FABP Reference Measurement Method: Reference Method)

An ELISA-based in vitro diagnostic product (Renapro (registered trademark) L-FABP test TMB) was used for a reference method.

### (First Reagent)

300 mmol/L buffer solution
0.1% BPF (manufactured by TOYOBO Co., Ltd., BPF-103)
300 mmol/L NaCl
390 mmol/L benzamidine hydrochloride
0.720 to 1.020 % Lipidure-BL103

### (Second Reagent)

5 mmol/L MOPS buffer solution (pH 7.0)
3.75 Abs/mL Clone L antibody-immobilized latex particle suspension^{(*)}
1.25 Abs/mL Clone 1 antibody-immobilized latex particle suspension^{(*)}
(*) Abs denotes the absorbance at 280 nm.

### (Standard Substance Diluent)

Phosphate buffer solution (pH 7.0)
0.1% BPF (manufactured by TOYOBO Co., Ltd., BPF-103)

### (Standard Solution)

The L-FABP standard substance was adjusted to desired concentrations by using the standard substance diluent to produce standard solutions.

### (Frozen-Thawed Urine)

Partial urine frozen and stored at -30 °C after collection was thawed only once and used for measurement.

### (Measurement Conditions of LTIA)

(1) Analyzing device: Hitachi 7170 Automatic Analyzer (manufactured by Hitachi High-Technologies Corporation)
(2) Sample amount and reagent amounts: 3 µL of sample, 150 µL of the first reagent, 50 µL of the second reagent
(3) Reaction time (reaction temperature): 5 minutes (37 °C) for the first reagent, 5 minutes (37 °C) for the second reagent
(4) Photometric point and photometric object: absorbance changes between immediately after addition of the second reagent and 5 minutes after the addition
(5) Measurement wavelength 570 nm/800 nm

### [Examples 5 to 9] Avoidance of Influence of Hemoglobin

The effect of avoiding an influence of hemoglobin was confirmed by using the buffer solutions shown in Table 3 for the first reagent.

### 1. Operation

### (1) Adjustment of Specimen

Interference check A plus (manufactured by SYSMEX CORPORATION) was adjusted to set the concentration of hemoglobin to 200 mg/dL. The adjusted Interference Check A Plus and the frozen-thawed urine were mixed in equal amounts to acquire a specimen in which hemoglobin coexisted at 100 mg/dL. For the specimen with the hemoglobin concentration of 0 mg/dL, the frozen-thawed urine was directly used.

### (2) Preparation of Calibration Curve

The standard solution was employed as a sample and L-FABP in the sample was measured by using the first reagent and the second reagent. From the measured absorbance, an absorbance of a 0 ng/mL L-FABP sample (blank absorbance) was subtracted to calculate the net absorbance. A calibration curve was prepared by plotting the L-FABP concentration on the x axis and the net absorbance on the y axis.

### (3) Examples 5 to 9

L-FABP in the specimen was measured by using the first reagent containing the buffer solutions of Table 3 and the second reagent.

### 2. Results

The L-FABP concentration was calculated by using the calibration curve of (2) from the absorbance measured in Examples 5 to 9. The calculated measurement value was divided by the measurement value at 0 mg/dL of hemoglobin to obtain a ratio (%) shown in Table 3.

**[Table 3]**

| | Buffer solution | Accuracy (%) |
|---|---|---|
| Ex.5 | BisTris pH6.85 | 110.9 |
| Ex.6 | HEPES pH7.0 | 102.1 |
| Ex.7 | HEPES pH7.5 | 112.5 |
| Ex.8 | TES pH7.0 | 109.1 |
| Ex.9 | TES pH7.5 | 106.9 |

| | | |
|---|---|---|
| Ex.: Example | | |

Variations in accuracy were within ±15% for all the buffer solutions containing BPF shown in Table 3 and it was confirmed that the influence of hemoglobin can be avoided. When the same test was performed on the buffer solution containing BPF at a concentration of 2.0%, the use of any of the buffer solutions enabled the avoidance of the influence of 500 mg/dL hemoglobin (results are not shown).

### [Example 10] Avoidance of Influence of Hemoglobin

The effect of avoiding an influence of hemoglobin was confirmed by using the buffer solution shown in Table 4 for the first reagent and 2.5 Abs/mL Clone L antibody-immobilized latex particle suspension and 2.5 Abs/mL Clone 1 antibody-immobilized latex particle suspension (Abs denotes the absorbance at 280 nm) for the second reagent. The other conditions are the same as those of Examples 2 to 9.

### 1. Operation

### (1) Adjustment of Specimen

Interference check A plus (manufactured by SYSMEX CORPORATION) was adjusted to set the concentration of hemoglobin to 200 mg/dL. The adjusted Interference Check A Plus and the frozen-thawed urine were mixed in equal amounts to acquire a specimen in which hemoglobin coexisted at 100 mg/dL. For the specimen with the hemoglobin concentration of 0 mg/dL, the frozen-thawed urine was directly used.

### (2) Preparation of Calibration Curve

The standard solution was employed as a sample and L-FABP in the sample was measured by using the first reagent and the second reagent. From the measured absorbance, an absorbance of a 0 ng/mL L-FABP sample (blank absorbance) was subtracted to calculate the net absorbance. A calibration curve was prepared by plotting the L-FABP concentration on the x axis and the net absorbance on the y axis.

### (3) Example 10

L-FABP in the specimen was measured by using the first reagent containing the buffer solution of Table 4 and the second reagent.

### 2. Results

The L-FABP concentration was calculated by using the calibration curve of (2) from the absorbance measured in Example 10. The calculated measurement value was divided by the measurement value at 0 mg/dL of hemoglobin to obtain the ratio (%) shown in Table 4.

**[Table 4]**

| | Buffer solution | Accuracy (°C) |
|---|---|---|
| Ex.10 | Tris pH7.0 | 114.3 |

| | | |
|---|---|---|
| Ex.: Example | | |

Variations in accuracy were within ±15% and it was confirmed that the influence of hemoglobin can be avoided.

### INDUSTRIAL APPLICABILITY

According to the present invention, the influence of hemoglobin on LTIA can be avoided by using BPF.

## Claims

1. An immunoassay for an analyte in a sample containing hemoglobin, comprising: reacting an analyte in a sample suspected of being present in the sample containing hemoglobin with an antibody binding to the analyte in the presence of a polypeptide consisting of amino acids No. 419 to No. 607 of the amino acid sequence of DnaK, a heat shock protein (HSP), derived from E. coli as set forth in SEQ ID NO: 1 or a polypeptide having at least 90 % sequence identity with the polypeptide.

2. The method according to claim 1, wherein the analyte in a sample containing hemoglobin is reacted with an antibody binding to the analyte also in the presence of one or more buffer components selected from HEPES, Bis-Tris, TES, and Tris.

3. The method according to claim 1 or 2, wherein the antibody is immobilized on an insoluble carrier.

4. The method according to claim 3, wherein the insoluble carrier is latex particles or metal colloid particles.

5. The method according to claim 4, wherein a particle agglutination measurement method is utilized.

6. The method according to any of claims 1 to 5, wherein the antibody is two or more monoclonal antibodies having recognition sites different from each other.

7. The method according to claim 6, wherein the two or more monoclonal antibodies having recognition sites different from each other are respectively immobilized on the latex particles, and wherein the analyte in the sample is detected by a latex turbidimetric immunoassay.

8. The method according to any of claims 1 to 7, wherein the sample is urine, whole blood, serum, or plasma.

9. The method according to any of claims 1 to 8, wherein the analyte is L-FABP (liver-type fatty acid binding protein).

10. A method of avoiding an influence of hemoglobin in a method of detecting an analyte in a sample containing hemoglobin with an antibody binding to the analyte, the method comprising:
reacting a sample suspected of containing the analyte with an antibody binding to the analyte
in the presence of a polypeptide consisting of amino acids No. 419 to No. 607 of the amino acid sequence of DnaK, a heat shock protein (HSP), derived from E. coli as set forth in SEQ ID NO: 1 or a polypeptide having at least 90 % sequence identity with the polypeptide.


SEQUENCE LISTING

<110>  SEKISUI MEDICAL CO., LTD.

<120>  Immunoassay and reagent therefor

<130>  15P00238

<150>  JP2015-035926
<151>  2015-02-25

<160>  1

<170>  PatentIn version 3.5

<210>  1
<211>  638
<212>  PRT
<213>  Escherichia coli

<220>
<221>  DnaK
<222>  (1)..(638)

<220>
<221>  BPF
<222>  (419)..(607)

<400>  1

Met Gly Lys Ile Ile Gly Ile Asp Leu Gly Thr Thr Asn Ser Cys Val
1               5                   10                  15

Ala Ile Met Asp Gly Thr Thr Pro Arg Val Leu Glu Asn Ala Glu Gly
            20                  25                  30

Asp Arg Thr Thr Pro Ser Ile Ile Ala Tyr Thr Gln Asp Gly Glu Thr
        35                  40                  45

Leu Val Gly Gln Pro Ala Lys Arg Gln Ala Val Thr Asn Pro Gln Asn
    50                  55                  60

Thr Leu Phe Ala Ile Lys Arg Leu Ile Gly Arg Arg Phe Gln Asp Glu
65                  70                  75                  80

Glu Val Gln Arg Asp Val Ser Ile Met Pro Phe Lys Ile Ile Ala Ala
                85                  90                  95

Asp Asn Gly Asp Ala Trp Val Glu Val Lys Gly Gln Lys Met Ala Pro
            100                 105                 110

Pro Gln Ile Ser Ala Glu Val Leu Lys Lys Met Lys Lys Thr Ala Glu
        115                 120                 125

Asp Tyr Leu Gly Glu Pro Val Thr Glu Ala Val Ile Thr Val Pro Ala
    130                 135                 140

Tyr Phe Asn Asp Ala Gln Arg Gln Ala Thr Lys Asp Ala Gly Arg Ile
145                 150                 155                 160

Ala Gly Leu Glu Val Lys Arg Ile Ile Asn Glu Pro Thr Ala Ala Ala
                165                 170                 175

Leu Ala Tyr Gly Leu Asp Lys Gly Thr Gly Asn Arg Thr Ile Ala Val
            180                 185                 190

Tyr Asp Leu Gly Gly Gly Thr Phe Asp Ile Ser Ile Ile Glu Ile Asp
        195                 200                 205

Glu Val Asp Gly Glu Lys Thr Phe Glu Val Leu Ala Thr Asn Gly Asp
    210                 215                 220

Thr His Leu Gly Gly Glu Asp Phe Asp Ser Arg Leu Ile Asn Tyr Leu
225                 230                 235                 240

Val Glu Glu Phe Lys Lys Asp Gln Gly Ile Asp Leu Arg Asn Asp Pro
                245                 250                 255

Leu Ala Met Gln Arg Leu Lys Glu Ala Ala Glu Lys Ala Lys Ile Glu
            260                 265                 270

Leu Ser Ser Ala Gln Gln Thr Asp Val Asn Leu Pro Tyr Ile Thr Ala
        275                 280                 285

Asp Ala Thr Gly Pro Lys His Met Asn Ile Lys Val Thr Arg Ala Lys
    290                 295                 300

Leu Glu Ser Leu Val Glu Asp Leu Val Asn Arg Ser Ile Glu Pro Leu
305                 310                 315                 320

Lys Val Ala Leu Gln Asp Ala Gly Leu Ser Val Ser Asp Ile Asp Asp
                325                 330                 335

Val Ile Leu Val Gly Gly Gln Thr Arg Met Pro Met Val Gln Lys Lys
            340                 345                 350

Val Ala Glu Phe Phe Gly Lys Glu Pro Arg Lys Asp Val Asn Pro Asp
        355                 360                 365

Glu Ala Val Ala Ile Gly Ala Ala Val Gln Gly Gly Val Leu Thr Gly
    370                 375                 380

Asp Val Lys Asp Val Leu Leu Leu Asp Val Thr Pro Leu Ser Leu Gly
385                 390                 395                 400

Ile Glu Thr Met Gly Gly Val Met Thr Thr Leu Ile Ala Lys Asn Thr
                405                 410                 415

Thr Ile Pro Thr Lys His Ser Gln Val Phe Ser Thr Ala Glu Asp Asn
            420                 425                 430

Gln Ser Ala Val Thr Ile His Val Leu Gln Gly Glu Arg Lys Arg Ala
        435                 440                 445

Ala Asp Asn Lys Ser Leu Gly Gln Phe Asn Leu Asp Gly Ile Asn Pro
    450                 455                 460

Ala Pro Arg Gly Met Pro Gln Ile Glu Val Thr Phe Asp Ile Asp Ala
465                 470                 475                 480

Asp Gly Ile Leu His Val Ser Ala Lys Asp Lys Asn Ser Gly Lys Glu
                485                 490                 495

Gln Lys Ile Thr Ile Lys Ala Ser Ser Gly Leu Asn Glu Asp Glu Ile
            500                 505                 510

Gln Lys Met Val Arg Asp Ala Glu Ala Asn Ala Glu Ala Asp Arg Lys
        515                 520                 525

Phe Glu Glu Leu Val Gln Thr Arg Asn Gln Gly Asp His Leu Leu His
    530                 535                 540

Ser Thr Arg Lys Gln Val Glu Glu Ala Gly Asp Lys Leu Pro Ala Asp
545                 550                 555                 560

Asp Lys Thr Ala Ile Glu Ser Ala Leu Thr Ala Leu Glu Thr Ala Leu
                565                 570                 575

Lys Gly Glu Asp Lys Ala Ala Ile Glu Ala Lys Met Gln Glu Leu Ala
            580                 585                 590

Gln Val Ser Gln Lys Leu Met Glu Ile Ala Gln Gln Gln His Ala Gln
        595                 600                 605

Gln Gln Thr Ala Gly Ala Asp Ala Ser Ala Asn Asn Ala Lys Asp Asp
    610                 615                 620

Asp Val Val Asp Ala Glu Phe Glu Glu Val Lys Asp Lys Lys
625                 630                 635